# EUROPEAN PATENT SPECIFICATION

(11) **EP 2 679 670 B1**
(45) Date of publication and mention of the grant of the patent: **05.10.2016**
(21) Application number: 12174200.1
(22) Date of filing: 28.06.2012
(51) Int. Cl.: C12N 5/00, C12N 5/077

(54) **Biomimetic cell culture support**
Biomimetische Zellkulturstütze
Support de culture cellulaire biomimétique

(43) Date of publication of application: 01.01.2014
(73) Proprietor: Parak, Wolfgang, 35037 Marburg (DE); Mahmoudi, Morteza, Teheran (IR); Sharifi, Shahriar, 3814 CC Amersfoort (NL)
(72) Inventor: Parak, Wolfgang, 35037 Marburg (DE); Mahmoudi, Morteza, Tehran (IR)
(74) Representative: Huebner, Stefan Rolf

(56) References cited:
- GUVENDIREN M ET AL: "The control of stem cell morphology and differentiation by hydrogel surface wrinkles", BIOMATERIALS, ELSEVIER SCIENCE PUBLISHERS BV., BARKING, GB, vol. 31, no. 25, 1 September 2010 (2010-09-01), pages 6511-6518, XP027102958, ISSN: 0142-9612 [retrieved on 2010-06-11]
- H. V. UNADKAT ET AL: "An algorithm-based topographical biomaterials library to instruct cell fate", PROCEEDINGS OF THE NATIONAL ACADEMY OF SCIENCES, vol. 108, no. 40, 4 October 2011 (2011-10-04), pages 16565-16570, XP055044041, ISSN: 0027-8424, DOI: 10.1073/pnas.1109861108
- K. A. KILIAN ET AL: "Geometric cues for directing the differentiation of mesenchymal stem cells", PROCEEDINGS OF THE NATIONAL ACADEMY OF SCIENCES, vol. 107, no. 11, 16 March 2010 (2010-03-16), pages 4872-4877, XP055044042, ISSN: 0027-8424, DOI: 10.1073/pnas.0903269107
- SUNGJUNE PARK ET AL: "Artificial Leaves via Reproduction of Hierarchical Structures by a Fast Molding and Curing Process", MACROMOLECULAR RAPID COMMUNICATIONS, vol. 33, no. 15, 11 May 2012 (2012-05-11), pages 1300-1303, XP055044043, ISSN: 1022-1336, DOI: 10.1002/marc.201200056

## Description

### Field of the invention

The invention relates to a cell culture support with a surface.

### Background of the invention

There are several known support structures for cultivating cells. The UK patent application GB 2 482 612 A describes a cell culture support for culturing mesenchymal stem cells with a surface comprising a plurality of wells, wherein the surface has a root mean square roughness (Rq) of 100 to 280 nm and a linear density of 1.6 to 3.0 per 1 µm length. The well shapes and the well opening sizes may be used to control the 3D structuring of mesenchymal stem cell aggregates and their sizes during the culturing in the well. The described cell culture support may induce mesenchymal stem cells to differentiate into tissue cells such as hyaline chondrocytes, adipocytes and osteoblasts.

The European patent application EP 0 684 309 A1 refers to a cell culture substrate comprising dried films of native fibrillar collagen produced by a method, in which collagen fibres are hydrolysed in acid, solubilised and reformed as gels on porous surfaces and under non-physiologic salt conditions to produce large fibres with the striations characteristic of collagen fibres found *in vivo.* Such cell cultures substrates enhance expression of barrier function in intestinal epithelial cells as compared to the collagen cell culture substrates of the prior art. Furthermore, intestinal epithelial cells cultured on the native fibrillar collagen substrates express barrier functions more rapidly even in the absence of differentiation inducing agents.

J. Lock et al. report in "nanomaterials enhance osteogenic differentiation of human mesenchymal stem cells similar to a short peptide of BMP-7", International Journal of Nanomedicine, 2011:6, pages 2769 to 2777, that nano-hydroxyapatite and nano-hydroxyapatite-PLGA composites provide a possibility to direct the adhesion and differentiation of human mesenchymal stem cells. The said composites promote osteogenic differentiation of human mesenchymal stem cells comparable with direct injection of a bone morphogenetic protein derived short peptide.

M. Guvendiren et al. describe in "The control of stem cell morphology and differentiation by hydrogel surface wrinkles", BIOMATERIALS, vol. 31, no. 25, 1 September 2010, pages 6511-6518, that wrinkled hydrogels can serve as a substrate for human mesenchymal stem cells (hMSC). In order to yield reproducible results, wrinkled hydrogels were produced as replica gels from wrinkled poly(2-hydroxyethyl methacrylate) (PHEMA) masters. Depending on the type of wrinkles that the masters show, hMSCs differentiated into an osteogenic phenotype or an adipogenic phenotype.

H. V. Unadkat et al. in "An algorithm-based topographical biomaterials library to instruct cell fate", PNAS, vol. 108, no. 40, 4 October 2011, pages 16565-16570, state that "unfortunately, nature does not prescribe the optimal surface topography for a given biomedical application". Therefore, the authors select 2176 distinct, randomly designed surface topographies, on which they cultivate human mesenchymal stromal cells (hMSC). Depending on the surface topography, where the individual cells were cultivated, proliferation and osteogenic differentiation were induced to different degrees.

K. A. Kilian et al. observe in "Geometric cues for directing the differentiation of mesenchymal stem cells", PNAS, vol. 107, no. 11, 16 March 2010, pages 4872-4877 that shape can be used to promote the differentiation of MSC to distinct lineages. To this end, cells were cultivated in flower and star shapes. The authors found that shapes promoting increased contractility led to preferential osteogenesis, when cells were exposed to a mixture of lineage cues. In contrast, cells in shapes that promote low contractility preferred to follow an adipogenic lineage.

Sungjune Park et al., "Artificial Leaves via Reproduction of Hierarchical Structures by a Fast Molding and Curing Process", Macromolecular Rapid Communications, vol. 33, no. 15, 11 May 2012, pages 1300-1303 describe a practical approach for the replication of hierarchical structures on a native leaf surface via a fast two step molding process combining both the fast durability to prevent shrinkage of cells and the strong resistance to chemical compounds for further applications. Static water contact angle measurements showed that the biomimetic surfaces exhibit wettability similar to the native leaves.

### Problem according to the invention

A goal of the present invention is to provide an improved cell culture support and an improved method to cultivate cells.

### Solution according to the invention

In order to solve the above problem, the invention teaches a cell culture support with a surface, wherein the structure of the surface is a positive or negative derivation of the surface structure of one cell or an association of cells as stated in claim 1. Furthermore, the problem is solved by the use of a cell culture support surface to cultivate cells, wherein the structure of the surface is a positive or negative derivation of the surface structure of one cell or an association of cells as described in claim 3. An additional solution is provided in claim 4 by a method for cultivating cells, wherein the cells are brought into close proximity to a cell culture support surface and wherein the structure of the surface is a positive or negative derivation of the surface structure of one cell or an association of cells. Claim 7 solves the problem by providing a method for producing a cell culture support, wherein the surface structure of one cell or an association of cells is recorded and a surface structure that resembles the recorded surface structure is reproduced onto the cell culture support. Moreover, the problem according to the invention is solved by claim 9, which discloses a method for cultivating cells in order to yield a specific cell type, wherein the cells are cultivated on a cell culture support, the surface structure of which is a positive or negative derivation of the surface structure of one cell or an association of the specific cell type.

In the case of the claims 1 to 4 and 9, the cell culture support surface structure is a positive derivation of the original surface structure. This means that topographical elements, which protrude from the original surface structure also protrude from the derived cell culture support surface structure. In other words, the yielded surface structure is a positive of the original surface structure. Alternatively, the cell culture support surface structure can be the negative shape of the original surface structure, such as can be the case with a cast obtained from the original surface structure. If the cell culture support surface structure is a positive then that positive imprints a shape onto the initial cells, which is similar to the shape that would be imprinted by the original surface structure. On the other hand, if the cell culture support surface structure is a negative of the original surface structure, the initial cells are forced into the shape of the original cells. Without prejudice, the inventors have obtained data to suggest that forcing an initial cell into the shape of an original cell will change the characteristics of the initial cell, which may ultimately lead to the initial cell assuming the same fate as the original cell. Advantageously, the cell culture support surface structure derived from a measurement of an original surface structure shows a high degree of resemblance to the original surface structure.

According to claim 7, a surface structure that resembles the recorded surface structure is reproduced onto the cell culture support. By recording the original surface structure and then reproducing it onto the cell culture support, changes can be made to the surface structure before it is reproduced. For example, a degree of abstraction can be introduced while still producing a cell culture support with the desired properties. Furthermore, errors in the recording can be corrected. The separation of recording and reproduction also makes it easier to mass-produce the cell culture support surface. For example, a small area can be recorded and its surface structure can then be reproduced by repeatedly applying it over a larger surface, much like a pavement of identical elements.

An association of cells according to the invention comprises at least two cells, preferably several cells, which may or may not be connected to each other. The connected cells in the association of cells may form a direct contact with each other and/or may be connected via extracellular components such as extracellular matrix proteins. The association of cells may contain cells of different types. When the surface structure of the cell culture support and the surface structure of the one cell or the association of cells resemble each other, it means that the two said surface structures have at least one, preferably two, more preferably three, most preferably five essential features, in which they are equal or show a high degree of similarity. As far as the feature is expressible as a numerical value, a high degree of similarity in a feature means that its value for a first surface structure is preferably within 50% and 150% preferably within 75% and 125%, preferably within 90% and 110%, preferably within 95% and 105%, preferably within 98% and 102%, preferably within 99% and 101%, preferably within 99.9% and 100.1% of its value of a second surface structure to be compared to the first surface structure.

Essential features of the said surface structures include roughness, cavity density, cavity dimensions, ratios of cavity dimensions and other topographical characteristics. Cavities according to the invention can be structures that contain a volume below as well as structures that contain a volume above the surface plane of the surface structure, if such a surface plane exists. In other words, cavities can contain raised and/or recessed portions. The scale of the surface structure according to the invention can be, when referring to a feature, a scale on the order of millimetres, micrometres and/or nanometres. The features of a surface structure can differ according to the scale applied. A surface structure can, for example, appear to be smooth on a millimetre scale yet contain numerous cavities on a micrometre scale.

Roughness can be measured in at least one of its key parameters such as the ones specified in the international standard ISO 25178 (the relevant portions of which are included in the present disclosure by reference) including, but not limited to the root mean square height of the surface, skewness of height distribution, kurtosis of height distribution, maximum height of peaks, maximum height of valleys, maximum height of the surface, arithmetical mean height of the surface, fastest decay auto-correlation rate, texture aspect ratio of the surface, texture direction of the surface, root mean square gradient of the surface, developed area ratio, surface bearing area ratio, height of surface bearing area ratio, peak extreme height, material volume at a given height, void volume at a given height, material volume of peaks, material volume of the core, void volume of the core, void volume of the valleys, density of peaks, arithmetic mean peak curvature, 10 point height, 5 point peak height, 5 point valley height, closed dales area, closed hills area, closed dales volume and closed hills volume. Cavity density is a measure for cavities present per area of the surface structure. Cavity dimensions can be determined by gauging the dimensions of the cavities along the x-, y- and z-axes of the surface structure or alternatively along such axes which are defined by the longest and/or smallest dimensions of the respective cavities. For example, the cavity dimensions can be measured along the length axis of the cavities - if a length axis exists - and two further axes perpendicular to the length axis. If the said surface structures display at least some degree of self-similarity, their Hausdorff dimension can be seen as a topographical characteristic. Another topographical characteristic is the direction, in which the length axes of the cavities point. The particular shape of the cavities, for example, a round, oval, square, rectangular, polygonal, star-like or spindle-shape, is also an essential feature.

Herein, the surface structure of the one cell or the association of cells, which surface structure resembles the cell culture support surface structure, is also described as the original surface structure. Accordingly, the said one cell and the said association of cells are referred to as the original cells. Cells which are cultivated on the cell culture support according to the invention are termed initial cells at the time when they are first placed on the cell culture support. The cells that develop from the initial cells while situated on the cell culture support are referred to as derived cells. When the original surface structure is recorded in a method to produce a cell culture support, it means that the original surface structure is measured so that at least one of its features is recorded.

If a cell culture support surface structure resembles a surface structure of one cell or an association of original cells of a specific type and initial cells are cultivated on the cell culture support surface structure, derived cells of the specific type can be yielded. When the original cells are of the same type as the derived cells, it means that original and derived cells share important characteristics. Such important characteristics include the cell shape or form and the cell surface structure on a micrometre and/or nanometre scale. Further important characteristics are the expression of one or several genes. Measurements of gene expression can be obtained by, for example, quantitative polymerase chain reaction, DNA sequencing or from a DNA microarray. Individual cell types can show a typical gene expression pattern in which a defined set of genes show certain expression level states. The expression level can take different states including no expression, low expression, intermediate expression and high expression. Preferably, the expression level of a gene is normalised to the expression level of a housekeeping gene. The threshold values that separate the expression level states depend on the measurement procedure applied. Gene expression patterns and corresponding threshold values are known to the person skilled in the art and can be used to identify a specific cell type. Furthermore, gene expression patterns of individual cell types can be obtained from public databases such as the Gene Expression Omnibus (R. Edgar et al., Gene Expression Omnibus: NCBI gene expression and hybridization array data repository in Nucleic Acids Res. 2002 Jan 1;30(1):207-10, the relevant portions of which are included in the present disclosure by reference).

The derived cells and the original cells are regarded as belonging to the same specific type when at least 50%, preferably at least 75%, more preferably at least 90% and most preferably 100% of the genes being part of the expression pattern of the specific type in question show the same expression level states.

Alternatively, the identification of the cell type can be carried out by measuring protein expression levels, for example using western blot, ELISA, flow cytometry of antibody stained cells, antibody microarrays, 2D gel electrophoresis or protein NMR. Whether original and derived cells are of the same specific type based on protein expression can be determined in analogy to the analysis based on gene expression levels as stated above.

When the derived cell is yielded from the initial cell, it means that the derived cell has remained unchanged or that it has changed. This change can be a differentiation, a dedifferentiation, or a "sideways" change, in which one differentiated cell may become another differentiated cell in a distinct differentiation path.

For the original cells in the association of cells to be said to be of the specific type, at least 0.1 %, preferably at least 1%, preferably at least 5%, preferably at least 10%, preferably at least 50%, preferably at least 70% and most preferably at least 90% of the original cells need to be of the specific type.

Yielding cells of the specific type means that at least some cells of the specific type are yielded, that is, at least 0.1 %, preferably at least 1%, preferably at least 5%, preferably at least 10%, preferably at least 50%, preferably at least 70% and most preferably at least 90% of cells of the specific type are yielded. Preferably more cells of the specific type are yielded than cultivation of the initial cells on an unstructured support structure in otherwise identical cell culture conditions would be yielded.

By providing a cell culture support with a surface structure, which mimics essential features of the surface structure of one cell or an association of cells, it is possible to cultivate cells in more natural surroundings. By recording a surface structure of one cell or an association of cells, it is possible to manufacture cell culture supports with surface structures that closely resemble the surface structure of the association of cells. When cells are cultivated to yield a specific cell type, it is advantageous to cultivate the cells on a surface structure, which resembles one cell or an association of the specific type. In that way, the physiologic milieu can be replicated more closely. The more natural cell culture environment may yield more biologically and medically relevant data as the results might be less distorted by artificial stimuli.

### Preferred embodiments of the invention

Preferred features of the invention, which may be applied alone or in combination are discussed below and in the dependent claims.

Measurements of the original surface structure can be obtained, for example, by using a non-contact procedure, including optical measurements, preferably using a laser, to determine the original surface structure. A non-contact method can yield high resolution structural data while it is often possible to leave the original surface structure intact. An atomic force microscope (AFM) can be employed in a non-contact procedure. In this case, the AFM may be placed in tapping mode, in which the AFM's cantilever is oscillated above the original surface structure without touching it and the cantilever's height above the original surface structure is determined by changes in its resonance frequency. The original surface structure may be coated with metal before the AFM measurement. In this way, data with a very high spatial resolution can often be obtained.

Alternatively, contact based techniques as for example stylus-like instruments can be used. Furthermore, an AFM can be used in a contact based procedure. To this end, the AFM can be placed in contact mode, in which the AFM's cantilever is touching the original surface structure and the height of the surface structure is determined by adjusting the cantilever such that the deflection of the cantilever remains constant. Contact based techniques are often less complex and may be implemented at a lower expense.

In a preferred embodiment according to the invention, the structure of the cell culture support surface resembles the surface structure of one cell or an association of cells of a specific cell type and cultivating cells on the cell culture support yields cells of the specific type. Without prejudice, the cell culture support according to the invention may force the initial cells into the shape of the original cells, which in turn may cause the initial cells to develop into derived cells of the cell type of the original cells. In this way, cells of the specific type may be yielded reliably.

In a preferred embodiment of the method for cultivating cells according to the invention, the cells to be cultivated are stem cells. Stem cells are cells which have not entirely differentiated into a cell type at the end of a differentiation path. Examples for stem cells are omnipotent stem cells, which can differentiate into any cell type, pluripotent stem cells, which can differentiate into many, but not all types of cells and multipotent stem cells, which can only differentiate into a limited number of cell types, namely those of a closely related family of cells. Cultivating stem cells on the cell culture support according to the invention carries the advantage that a large number of different cell types can be yielded. However, the invention is not limited to stem cells. In principle, any type of cell can be cultivated and/or yielded in the method for cultivating cells according to the invention. This applies in particular to such cells which can form a tissue, including, for example, fibroblasts, cardiac tissue cells and neuronal cells.

In a preferred method according to the invention, the cultivation on the cell culture support surface yields a specific cell type. When initial cells are cultivated on top of the cell culture support according to the invention, derived cells of the specific cell type may be yielded. This can be an easy way to produce cells of the specific type.

In a preferred embodiment of the invention, the recording is performed by the production of a cast from one cell or an association of cells. The cast can be obtained by pouring a liquid, which subsequently hardens, on the one cell or the association of cells. The liquid is preferably a cross-linkable polymer such as, for example, poly(methyl methacrylate (PMMA) or poly(ethylene glycol)-co-fumarate (PEGF). By employing the said substances, a high degree of resemblance of the surface structure of the cast with the surface structure of the one cell or the association of cells can be achieved.

### Brief description of the drawings

The invention is illustrated in greater detail with the aid of graphs, a microscopy image and schematic drawings.
- Fig. 1: shows cells cultivated on a cell culture support;
- Fig. 2: shows the results of quantitative polymerase chain reaction experiments and
- Fig. 3: shows the production, an atomic force microscopy image and a profile of a cast.

### Detailed description of the embodiments of the invention

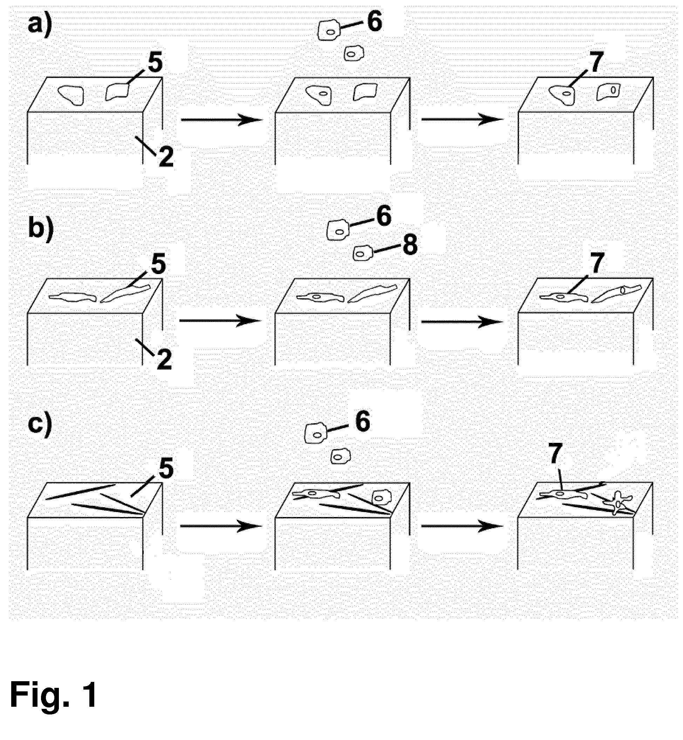

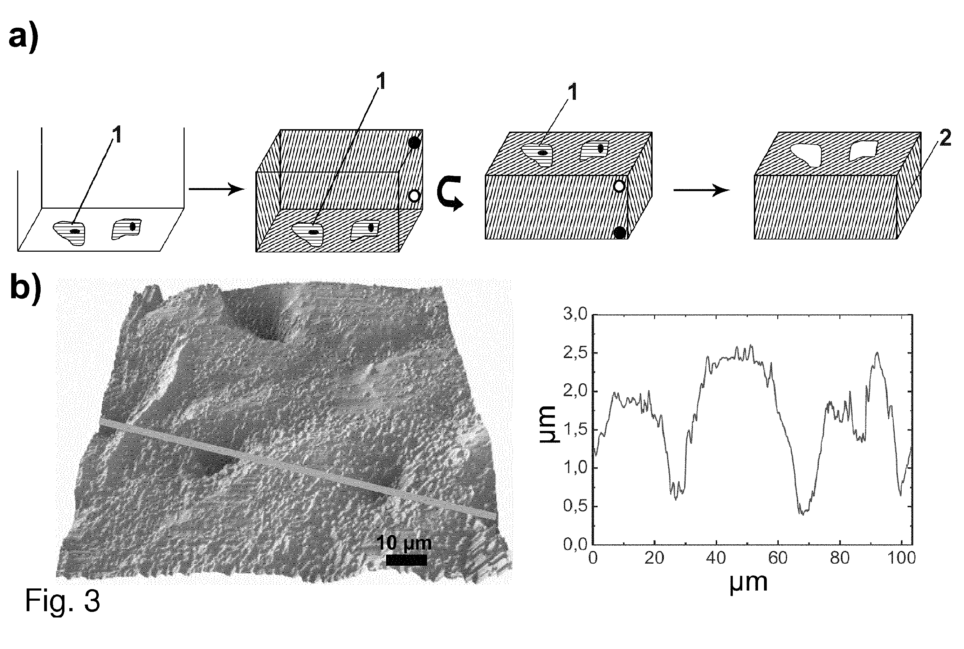

Chondrocytes were isolated from cartilage slices of white New Zealand rabbits. The isolated chondrocytes were cultivated on tissue culture polystyrene plates as a monolayer culture. One week after isolation, the chondrocytes in tissue culture had a spherical shape. Three weeks after isolation, the chondrocytes had dedifferentiated into spindle-shaped fibroblasts (figure 3a, first schematic drawing from the left). Subsequently, casts were obtained from both the spherical chondrocyte cultures as well as from the spindle-shaped fibroblasts. This was achieved by pouring silicone on top of the association of original cells 1 - which were, in this case, either spherical chondrocytes or spindle-shaped fibroblasts - contained in the tissue culture plates (figure 3a, second schematic drawing from the left). The silicone was selected for its ease of handling, its ability to reproduce surface structures on a nanoscale, the capability to come into close contact with the original cells 1 in culture, its transparency and rubbery, elastic property. The silicone used was polydimethyl siloxane (PDMS, brand name: Sylgard, which is room-temperature vulcanising and was obtained from Dow Corning, USA). After the silicone mass had cured on top of the original cells 1, it was removed from the cell culture plates (figure 3a, third schematic drawing from the left). Then the silicone was washed extensively with 1 M sodium hydroxide solution in order to remove cell debris and protein residue (figure 3a, fourth schematic drawing from the left). In this way, a negative cast 2 of spindle-shaped fibroblasts was acquired, demonstrated in figure 1 b. Furthermore, a negative cast 2 of spherical chondrocytes was obtained as shown in figure 1 a. Figure 3b shows an atomic force microscopy image and profile obtained from one of the said silicone casts 2 of spherical chondrocytes. The image demonstrates the formation of cellular 3D shapes on the silicone cast 2. The casts 2 were then used as cell culture supports 3 with a surface 4, which structure 5 resembled an association of original cells 1.

Initial cells 6, particularly adipocyte derived mesenchymal stem cells (ADSCs) were cultivated on the spherical chondrocyte cast 2 (shown in figure 1a, left) and on the spindle-shaped fibroblast cast 2 (shown in figure 1 b, left). It was confirmed that the ADSCs attached themselves to the cell culture support surface 3 of the silicone. After 1 week, derived cells 7 were obtained from the initial cells 6. When comparing the derived cells 7 cultivated on the two different silicone casts 2 by optical, fluorescence and atomic force microscopy, it was found that the ADSCs had transformed into two morphologically different cell types. When cultivated on the spherical chondrocyte derived silicone cast 2, the ADSCs assumed a spherical shape (figure 1a, right). In contrast, the ADSCs cultivated on the spindle-shaped fibroblast derived cast 2 developed into derived cells 7 that had a spindle-like configuration (figure 1 b, right).

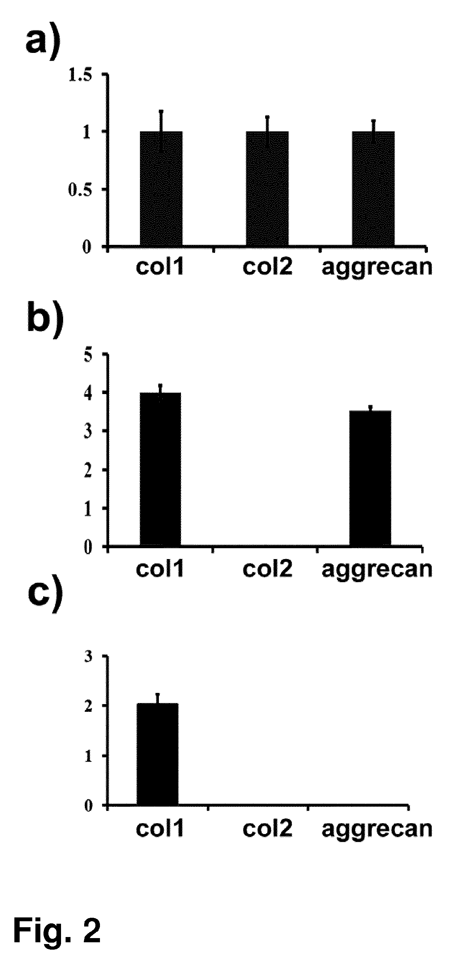

To confirm the morphological findings on a molecular level, gene expression levels of the derived cells 7 cultivated on the silicone casts 2 were measured. To this end, ADSCs were cultivated for 5 weeks on the silicone casts obtained from chondrocytes and fibroblasts as well as on regular polystyrene tissue culture plates (figure 1 c). RNA was isolated from the derived cells 7 and quantitative polymerase chain reaction was carried out. Specifically, the expression of the genes aggrecan, col1 and col2 was measured. Results are displayed in figure 2 normalised to the expression level of the housekeeping gene glyceraldehyde-3-phosphate dehydrogenase. The ADSCs cultivated on chondrocyte derived silicone casts 2 expressed high levels of the chondrocyte markers aggrecan, col1 and col2 (figure 2a). The ADSCs on the fibroblast derived casts 2 also expressed high levels of aggrecan and col1, however, there was no expression of col2 (figure 2b). Furthermore, while the ADSCs cultivated on the regular polystyrene plates expressed high levels of col1, neither col2 nor aggrecan was expressed (figure 2c). This shows that the gene expression levels differ, depending on what the cell culture surface 4 was derived from. Moreover, the expression also differs from derived cells 7 cultivated on the "neutral" polystyrene plate. Particularly striking is the expression of the chondrocyte marker col2, which was only present on the ADSCs cultivated on casts derived from chondrocytes.

Without prejudice, the inventors conclude that cell culture surfaces 4 derived from cell cultures can drive the differentiation of cells 8. The inventors presume - without prejudice - a mechanism that can be described as shape-induced differentiation. In this mechanism, when an initial cell is shaped by external force applied through the cell culture support, changes to the cell signalling pathways occur, forcing the initial cells to adopt the same type as the original cells.

The features disclosed in the above description, the claims and the figures can be of importance both individually and in any desired combination for the realisation of the invention in its various embodiments.

### Protocols

### Isolation of Stem Cells and Chondrocytes

The isolation of ADSCs and chondrocytes was performed according to the following procedure: rabbit articular cartilage (White New Zealand rabbits) were sliced and were digested in trypsin-EDTA solution (0.25%, Sigma, USA) for 1 h and collagenase type II (0.08 mg/mL, Sigma, USA) solution for 24 hours at 37°C. In order to isolate the ADSCs, the adipose tissue was harvested from the upper part of the intestine and digested in collagenase type I (0.02 mg/ml, Sigma, USA) for 1 hour in an incubator. ADSCs were cultivated in 24 well tissue culture polystyrene plates at a density of 3×10⁴ cells per well.

### Stem Cell Culture Protocol on the Silicone Substrates

ADSCs were cultivated on the casts at a density of 3×10⁴ cells per cast for 1 week. The morphology of the cells was assessed by optical examination with and without fluorescence staining of microtubules. The cells were fixed in paraformaldehyde (4%, Sigma, USA) for 15 minutes and permeabilised in triton x100 (0.01%, Sigma, USA) for 10 minutes. Actin filaments were stained with FITC conjugated phalloidin (Sigma, USA) for 45 minutes in the dark and visualised by fluorescence microscopy (Zeiss, Axioscope, Germany). Cell nuclei were stained with Hoechst 33258 dye (Sigma, USA) and merged with actin staining.

### RNA isolation and processing

The RNeasy MiniKit (74104, QIAGEN, Germany) was utilised for RNA extraction of the cultivated cells according to manufacturer instructions. The concentration of cellular RNA was quantified by determining the absorbance maximum at 260-nm wavelength in a spectrophotometer (Eppendorf, Germany). cDNA was obtained by mixing 1 µg of total RNA and 20 µl of reaction mixture including 4 µl PCR buffer (15X), 2 µl dNTPs (20 mM, Roche, Germany), 1 µl 10 pmol/µl random hexamer (N6; Roche, Germany), 2 µl deionised sterile H₂O and 1 µl reverse transcriptase (200 U/µl; Fermentase, Russia). Finally, the mixture was kept at 42 ° C for 45 min and then incubated at 90 ° C for 5 min.

### Real time PCR analysis

The real-time PCR was performed in an ABI 7300 real-time PCR system (Applied Biosystems) with SYBR Green PCR master mix (Applied Biosystem) according to manufacturer instructions.

### List of reference numbers

- 1: original cell
- 2: cast
- 3: cell culture supports
- 4: cell culture supports surface
- 5: cell culture supports surface structure
- 6: initial cells
- 7: derived cells
- 8: cells

## Claims

1. Cell culture support (3) with a surface (4), **characterised in that** the structure (5) of the surface (4) is a positive or negative derivation of the surface structure of one cell or an association of cells (8), wherein the structure (5) of the surface (4) is derived by recording the surface structure of the one cell or the association of cells of the specific cell type and reproducing a surface structure that resembles the recorded surface structure onto the cell culture support.

2. Cell culture support (3) according to one of the preceding claims, **characterised in that** the structure (5) of the surface (4) is a positive of negative derivation of the surface structure of one cell or an association of cells (8) of a specific cell type and cultivating cells (8) on the cell culture support (3) yields cells (8) of the specific type.

3. Use of a cell culture support surface (4) to cultivate cells (8), **characterised in that** the structure (5) of the surface (4) is a positive or negative derivation of the surface structure of one cell or an association of cells (8), wherein the structure (5) of the surface (4) is derived by recording the surface structure of the one cell or the association of cells of the specific cell type and reproducing a surface structure that resembles the recorded surface structure onto the cell culture support.

4. Method for cultivating cells (8), wherein the cells (8) are brought into close proximity to a cell culture support surface (4), **characterised in that** the structure (5) of the surface (4) is a positive or negative derivation of the surface structure of one cell or an association of cells (8), wherein the structure (5) of the surface (4) is derived by recording the surface structure of the one cell or the association of cells of the specific cell type and reproducing a surface structure that resembles the recorded surface structure onto the cell culture support.

5. Method according to claim 4, **characterised in that** the cells (8) to be cultivated are stem cells.

6. Method according to claim 4 or 5, **characterised in that** the cultivation on the cell culture support surface (4) yields a specific cell type.

7. Method according to claim 6, **characterised in that** the structure (5) of the surface (4) is a positive or negative derivation of the surface structure of one cell or an association of cells (8) of the specific cell type.

8. Method for producing a cell culture support (3), **characterised by** the following steps:
recording the surface structure (5) of one cell or an association of cells (8);
reproducing a surface structure (5) that resembles the recorded surface structure onto the cell culture support (3).

9. Method for producing a cell culture support (3) according to claim 8, **characterised in that** the recording is performed by the production of a cast (2) from one cell or an association of cells (8).

## Patentansprüche

1. Zellkulturstütze (3) mit einer Oberfläche (4), **dadurch gekennzeichnet, dass** die Struktur (5) der Oberfläche (4) eine positive oder negative Ableitung der Oberflächenstruktur von einer Zelle oder eines Zellverbandes (8) ist, wobei die Struktur (5) der Oberfläche (4) durch Aufnehmen der Oberflächenstruktur der einen Zelle oder des Zellverbandes des spezifischen Zelltyps und Reproduzieren einer Oberflächenstruktur, die der aufgenommenen Oberflächenstruktur auf der Zellkulturstütze ähnelt, abgeleitet wird.

2. Zellkulturstütze (3) gemäß einem der vorstehenden Ansprüche, **dadurch gekennzeichnet, dass** die Struktur (5) der Oberfläche (4) eine positive oder negative Ableitung der Oberflächenstruktur von einer Zelle oder eines Zellverbandes (8) von einem spezifischen Zelltyp ist und kultivierte Zellen (8) auf der Zellkulturstütze (3) Zellen (8) von dem spezifischen Typ ergeben.

3. Verwendung einer Oberflächenstruktur (4) um Zellen (8) zu kultivieren, **dadurch gekennzeichnet, dass** die Struktur (5) der Oberfläche (4) eine positive oder negative Ableitung der Oberflächenstruktur der einen Zelle oder des Zellverbandes (8) ist, wobei die Struktur (5) der Oberfläche (4) durch Aufnehmen der Oberflächenstruktur der einen Zelle oder des Zellverbandes des spezifischen Zelltyps und Reproduzieren der Oberflächenstruktur, die der aufgenommenen Oberflächenstruktur auf der Zellkulturstütze ähnelt, abgeleitet wird.

4. Verfahren zum Kultivieren von Zellen (8), wobei die Zellen (8) in einen Nahbereich der Zellkulturstützfläche (4) gebracht werden, **dadurch gekennzeichnet, dass** die Struktur (5) der Oberfläche (4) eine positive oder negative Ableitung der Oberflächenstruktur der einen Zelle oder des Zellverbandes (8) ist, wobei die Struktur (5) der Oberfläche (4) durch Aufnehmen der Oberflächenstruktur der einen Zelle oder des Zellverbandes des spezifischen Zelltyps und Reproduzieren einer Oberflächenstruktur, die der aufgenommenen Oberflächenstruktur auf der Zellkulturstütze ähnelt, abgeleitet wird.

5. Verfahren nach Anspruch 4, **dadurch gekennzeichnet, dass** die zu kultivierenden Zellen (8) Stammzellen sind.

6. Verfahren nach Anspruch 4 oder 5, **dadurch gekennzeichnet, dass** die Kultur auf der Zellkulturstützoberfläche (4) einen spezifischen Zelltyp ergibt.

7. Verfahren gemäß Anspruch 6, **dadurch gekennzeichnet, dass** die Struktur (5) auf der Oberfläche (4) eine positive oder negative Ableitung der Oberflächenstruktur der einen Zelle oder eines Zellverbandes (8) des spezifischen Zelltyps ist.

8. Verfahren zum Erzeugen einer Zellkulturstütze (3), **gekennzeichnet durch** die folgenden Schritte: Aufnehmen der Oberflächenstruktur (5) von einer Zelle oder eines Zellverbandes (8); Reproduzieren einer Oberflächenstruktur (5), die der aufgenommenen Oberflächenstruktur auf der Zellkulturstütze (3) ähnelt.

9. Verfahren zum Erzeugen einer Zellkulturstütze (3) nach Anspruch 8, **dadurch gekennzeichnet, dass** das Aufnehmen durch Erzeugen eines Abgusses (2) von einer Zelle oder eines Zellverbandes (8) ausgeführt wird.

## Revendications

1. Support pour culture de cellules (3) avec une surface (4), **caractérisé en ce que** la structure (5) de la surface (4) est une obtention positive ou négative de la structure de surface d'une unique cellule ou d'une association de cellules (8), dans laquelle la structure (5) de la surface (4) est obtenue en enregistrant la structure de surface de la cellule unique ou de l'association de cellules du type de cellules spécifique et la reproduction d'une structure de surface qui ressemble à la structure de surface enregistrée sur le support pour culture de cellules.

2. Support pour culture de cellules (3) avec une surface (4), **caractérisé en ce que** la structure (5) de la surface (4) est une obtention positive ou négative de la structure de surface d'une unique cellule ou d'une association de cellules (8) d'un type de cellules spécifique et la culture de cellules (8) sur le support pour culture de cellules (3) produit des cellules (8) du type spécifique.

3. Utilisation d'un support pour culture de cellules (4) afin de cultiver des cellules (8), **caractérisée en ce que** la structure (5) de la surface (4) est une obtention positive ou négative de la structure de surface d'une unique cellule ou d'une association de cellules (8), dans laquelle la structure (5) de la surface (4) est obtenue en enregistrant la structure de surface de la cellule unique ou de l'association de cellules du type de cellules spécifique et la reproduction d'une structure de surface qui ressemble à la structure de surface enregistrée sur le support pour culture de cellules.

4. Méthode de culture de cellules (8), dans laquelle les cellules (8) sont mises à proximité immédiate d'une surface de support pour culture de cellules afin de cultiver des cellules (4), **caractérisée en ce que** la structure (5) de la surface (4) est une obtention positive ou négative de la structure de surface d'une unique cellule ou d'une association de cellules (8), dans laquelle la structure (5) de la surface (4) est obtenue en enregistrant la structure de surface de la cellule unique ou de l'association de cellules du type de cellules spécifique et la reproduction d'une structure de surface qui ressemble à la structure de surface enregistrée sur le support pour culture de cellules.

5. Méthode selon la revendication 4, **caractérisée en ce que** les cellules (8) à cultiver sont des cellules-souches.

6. Méthode selon les revendications 4 ou 5, **caractérisée en ce que** la culture sur la surface de support pour culture de cellule (4) produit un type de cellule spécifique.

7. Méthode selon la revendication 6, **caractérisée en ce que** la structure (5) de la surface (4) est une obtention positive ou négative de la structure de surface d'une cellule ou d'une association de cellule (8) du type de cellule spécifique.

8. Méthode de production d'un support pour culture de cellule (3), **caractérisée par** les étapes suivantes :
l'enregistrement de la structure de surface (5) d'une unique cellule ou d'une association de cellules (8) ;
la reproduction d'une structure de surface (5) qui ressemble à la structure de surface enregistrée sur le support pour culture de cellules (3).

9. Méthode de production d'un support pour culture de cellule (3) selon la revendication 8, **caractérisée en ce que** l'enregistrement est réalisé par la production d'un moulage (2) provenant d'une unique cellule ou d'une association de cellules (8).
